# EUROPÄISCHE PATENTSCHRIFT

(11) **EP 1 856 538 B1**
(45) Veröffentlichungstag und Bekanntmachung des Hinweises auf die Patenterteilung: **13.05.2009**
(21) Anmeldenummer: 06723243.9
(22) Anmeldetag: 06.03.2006
(51) Int. Cl.: G01N 33/68, G01N 33/569

(54) **BESTIMMUNG VON KURZKETTIGER SRL-ALKOHOLDEHYDROGENASE (DHRS4) ALS BIOMARKER FÜR ENTZÜNDUNGEN UND INFEKTIONEN**
DETERMINATION OF SHORT-CHAIN SRL-ALCOHOL DEHYDROGENASE (DHRS4) AS BIOMARKER OF INFLAMMATIONS AND INFECTIONS
DÉTERMINATION D'UNE SRL-ALCOOL DÉSHYDROGÉNASE (DHRS4) À COURTE CHAÎNE COMME BIOMARQUEUR POUR LES INFLAMMATIONS ET INFECTIONS

(30) Priorität: 11.03.2005 DE 102005011421
(43) Veröffentlichungstag der Anmeldung: 21.11.2007
(73) Patentinhaber: BRAHMS Aktiengesellschaft, 16761 Hennigsdorf (DE)
(72) Erfinder: BERGMANN, Andreas, 12351 Berlin (DE); STRUCK, Joachim, 13465 Berlin (DE); ÜHLEIN, Monika, 10407 Berlin (DE)
(74) Vertreter: Andrae, Steffen
(86) Internationale Anmeldenummer: PCT/EP2006/002043
(87) Internationale Veröffentlichungsnummer: WO 2006/094747

(56) Entgegenhaltungen:
- WO-A-20/04003162
- US-A1- 2002 160 392

### SEQUENCE LISTING

<110> B.R.A.H.M.S Aktiengesellschaft
<120> Bestimmung von kurzkettiger SRL-Alkoholdehydrogenase (DHRS4) als Biomarker für Entzündungen und Infektionen
<130> 4514 PCT
<150> DE 102005011421.0
   <151> 2005-03-11
<160> 5
<170> PatentIn version 3.2
<210> 1
   <211> 278
   <212> PRT
   <213> Homo sapiens
<400> 1
   Met His Lys Ala Gly Leu Leu Gly Leu Cys Ala Arg Ala Trp Asn Ser
   1               5                   10                  15
   Val Arg Met Ala Ser Ser Gly Met Thr Arg Arg Asp Pro Leu Ala Asn
               20                  25                  30
   Lys Val Ala Leu Val Thr Ala Ser Thr Asp Gly Ile Gly Phe Ala Ile
           35                  40                  45
   Ala Arg Arg Leu Ala Gln Asp Gly Ala His Val Val Val Ser Ser Arg
       50                  55                  60
   Lys Gln Gln Asn Val Asp Gln Ala Val Ala Thr Leu Gln Gly Glu Gly
   65                  70                  75                  80
   Leu Ser Val Thr Gly Thr Val Cys His Val Gly Lys Ala Glu Asp Arg
                   85                  90                  95
   Glu Arg Leu Val Ala Thr Ala Val Lys Leu His Gly Gly Ile Asp Ile
               100                 105                 110
   Leu Val Ser Asn Ala Ala Val Asn Pro Phe Phe Gly Ser Ile Met Asp
           115                 120                 125
   Val Thr Glu Glu Val Trp Asp Lys Thr Leu Asp Ile Asn Val Lys Ala
       130                 135                 140
   Pro Ala Leu Met Thr Lys Ala Val Val Pro Glu Met Glu Lys Arg Gly
   145                 150                 155                 160
   Gly Gly Ser Val Val Ile Val Ser Ser Ile Ala Ala Phe Ser Pro Ser
   165 170 175
   Pro Gly Phe Ser Pro Tyr Asn Val Ser Lys Thr Ala Leu Leu Gly Leu
   180 185 190
   Thr Lys Thr Leu Ala Ile Glu Leu Ala Pro Arg Asn Ile Arg Val Asn
   195 200 205
   Cys Leu Ala Pro Gly Leu Ile Lys Thr Ser Phe Ser Arg Met Leu Trp
   210 215 220
   Met Asp Lys Glu Lys Glu Glu Ser Met Lys Glu Thr Leu Arg Ile Arg
   225 230 235 240
   Arg Leu Gly Glu Pro Glu Asp Cys Ala Gly Ile Val Ser Phe Leu Cys
   245 250 255
   Ser Glu Asp Ala Ser Tyr Ile Thr Gly Glu Thr Val Val Val Gly Gly
   260 265 270
   Gly Thr Pro Ser Arg Leu
   275
<210> 2
   <211> 13
   <212> PRT
   <213> Synthetic
<400> 2
   Cys Met Ala Ser Ser Gly Met Thr Arg Arg Asp Pro Leu
   1 5 10
<210> 3
   <211> 20
   <212> PRT
   <213> Synthetic
<400> 3
   Cys Leu Ala Pro Gly Leu Ile Lys Thr Ser Phe Ser Arg Met Leu Trp
   1 5 10 15
   Met Asp Lys Glu
   20
<210> 4
   <211> 19
   <212> PRT
   <213> Synthetic
<400> 4
   Cys Glu Glu Ser Met Lys Glu Thr Leu Arg Ile Arg Arg Leu Gly Glu
   1               5                   10                  15
   Pro Glu Asp
<210> 5
   <211> 23
   <212> PRT
   <213> Synthetic
<400> 5
   Cys Ser Glu Asp Ala Ser Tyr Ile Thr Gly Glu Thr Val Val Val Gly
   1               5                   10                  15
   Gly Gly Thr Pro Ser Arg Leu
               20

## Beschreibung

Die vorliegende Erfindung betrifft Verfahren zum diagnostischen Nachweis und für die Verlaufsprognose sowie die Verlaufs- und Therapiekontrolle von Entzündungen und Infektionen, bei denen man einen für die angegebenen Indikationen neuartigen Biomarker bestimmt. Insbesondere betrifft die Erfindung Verfahren der genannten Art, bei denen die diagnostizierten Entzündungen und Infektionen Teil des komplexen Verlaufs eines septischen Krankheitsgeschehens (systemischer Entzündungen infektiöser Ätiologie; Sepsis) sind.

In der folgenden Beschreibung werden dabei Begriffe wie "Diagnostik" oder "diagnostisch" grundsätzlich als vereinfachende Oberbegriffe verwendet, die, wenn sich aus dem Zusammenhang nichts anderes ergibt, auch speziellere Differenzialdiagnostik und Anwendungen zur Prognostik/Frühprognostik und Verlaufs- und Therapiekontrolle der diskutierten Erkrankungen einschließen sollen.

Die vorliegende Erfindung hat ihren Ausgangspunkt in intensiven Forschungsarbeiten der Anmelderin im Zusammenhang mit weiteren Verbesserungen der Diagnose und Therapie von Sepsis.

Zwischen Sepsis und Entzündungen besteht ein wissenschaftlicher Sach- und Definitions-Zusammenhang. Als Entzündungen (Inflammationen) werden ganz allgemein bestimmte physiologische Schutzreaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet.

Wenn Entzündungen Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, können die Entzündungen zum eigentlichen Krankheitsgeschehen werden, die dann, wenn die für Entzündungsreaktionen typischen physiologischen Vorgänge außer Kontrolle geraten, wie bei SIRS und Sepsis, sogar zu einer akuten Lebensbedrohung werden können.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den gegenwärtigen Kenntnissen über das Auftreten und die mögliche Rolle einzelner Gruppen endogener entzündungsspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis, und damit auch die anerkannten Definitionen, in den letzten Jahren gewandelt haben und verfeinert wurden, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird, sowie insbesondere auch auf Mitchell M. Levy et al., "2001 SCCM/ESICM/ACCP/ATS/SIS International Sepsis Definition Conference", in: Crit Care Med 2003, Vol. 31, No.4, 1250-1256. Zur Bedeutung des Krankheitsbilds "schwere Sepsis" wird ferner verwiesen auf Niels C. Riedemann et al., The enigma of sepsis, J.Clin.Invest. 112:460-467 (2003). Eine jüngere Zusammenfassung der Kriterien und Definitionen für eine Sepsis und eng verwandte Krankheitsbilder findet sich auch unter http://www.talessin.de/scripte/medizin/sepsis1.html. Im Rahmen der vorliegenden Anmeldung wird der Begriff Sepsis in einem umfassenden Sinne, der insbesondere Sepsis, schwere Sepsis und Septischen Schock umfaßt, in Anlehnung an die Definitionen, wie sie den genannten Veröffentlichungen entnommen werden können, für septische Krankheitsbilder von schwer erkrankten Patienten auf Intensivstationen verwendet.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden.

Dem genannten Wandel des Sepsis-Verständnisses entsprechen Veränderungen der diagnostischen Ansätze. So wurde der direkte Nachweis bakterieller Erreger durch komplexe Überwachungen von Laborparametern und hämodynamischen Parametern unter Anwendung computergestützter sog. Score-Systeme (z.B. APACHE II SCORE; APACHE steht für "Acute Physiology and Chronic Health Evaluation"; vgl. G.Pilz et al., Krankenpflege-Journal 29 (1991), S.483-492 oder die Einleitung des Patents DE 42 27 454 C1) sowie in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen eignen sich dabei für diagnostische Zwecke insbesondere solche, deren Auftreten sehr spezifisch für Sepsis bzw. bestimmte Phasen einer Sepsis ist, deren Konzentrationen sich drastisch und diagnostisch signifikant verändern und die außerdem die für Routinebestimmungen erforderlichen Stabilitäten, insbesondere auch *ex vivo*, aufweisen und hohe Konzentrationswerte erreichen. Für diagnostische Zwecke steht dabei die zuverlässige Korrelation von Krankheitsgeschehen (Sepsis) mit dem jeweiligen Biomarker im Vordergrund, ohne dass dessen Rolle in der komplexen Kaskade der am Sepsisgeschehen beteiligten endogenen Substanzen stets im einzelnen bekannt sein muss. Es besteht jedoch ein zunehmendes Interesse daran, neue besondere Biomarker zu ermitteln, die im Sinne einer "Stratifizierung" (auch) eine Zuordnung der Sepsispatienten zu Gruppen mit verwandten Krankheitsursachen oder einem ähnlichen zu erwarteten Krankheitsverlauf ermöglichen, so dass aus dem Spektrum der möglichen therapeutischen Maßnahmen die geeignetsten zur Anwendung gebracht werden können. Es kann in diesem Zusammenhang ergänzend verwiesen werden auf John C. Marshall et al., Crit Care Med 2003, Vol 31, No.5, 1560-1567.

Eine etablierte, als Sepsis-Biomarker besonders geeignete endogene Substanz ist Procalcitonin (PCT). Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zur Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen.

Eine aktuelle Diskussion der Verwendung von Biomarkern, einschließlich des Biomarkers PCT, im Rahmen der Sepsisdiagnose findet sich auch in dem Review von Shawn D. Carrigan et al., "Toward Resolving the Challenges of Sepsis Diagnosis" in: Clinical Chemistry 50:8, August 2004, 1301-14.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik bzw. Stratifizierung zu liefern vermögen.

Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Sepsisgeschehen beteiligt sind, bekannt ist.

Erste Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439 der Anmelderin. Dort wird gezeigt, dass bei Sepsis nicht nur die Konzentration des Prohormons Procalcitonin erhöht ist, sondern auch für andere Substanzen, die zu den Peptid-Prohormonen gerechnet werden können oder die Fragmente solcher Prohormone darstellen und eine für solche Prohormone typische Immunreaktivität aufweisen, signifikant erhöhte Konzentrationen beobachtet werden können.

Die vorliegende Anmeldung ist Ergebnis eines anderen fruchtbaren, rein experimentellen Ansatzes für die Suche nach weiteren sepsisspezifischen Biomolekülen. Dieser beruht darauf, dass man durch Verabreichung eines Endotoxins oder durch Infektion mit Bakterien bei Primaten (Pavianen) einen als künstliche Sepsis bezeichenbaren Krankheitszustand auslöst und dann durch Vergleich der Gelelektrophorese-Proteinspotmuster von endotoxinbehandelten und von unbehandelten Pavianen endogene Substanzen von peptischer bzw. proteinischer Natur ermittelt, die nur bei den "septischen" Pavianen gefunden werden und die daher potentielle sepsisspezifische Biomarker darstellen. Das Primatenmodell wurde dabei aufgrund der sehr großen Ähnlichkeit der Physiologie von Primaten und Menschen und der hohen Kreuzreaktivität mit vielen therapeutischen und diagnostischen humanen Reagenzien gewählt.

Wie im experimentellen Teil älterer Patentanmeldungen der Anmelderin genauer ausgeführt wird, wird dabei nach experimenteller Auslösung einer künstlichen Sepsis in Pavianen durch Endotoxinverabreichung (LPS aus Salmonella Typhimurium) und 2D-gelektrophoretischer Aufarbeitung von Gewebe der behandelten Tiere eine Anzahl von nur bei den behandelten Tieren identifizierbaren Proteinspots gefunden. Die den Spots entsprechenden proteinischen Produkte werden aus dem Elektrophoresegel isoliert und massenspektrometrisch (v.a. mittels Tandem-Massenspektrometrie) untersucht.

Nach dem genannten Verfahren wurden, wie in älteren deutschen und europäischen Patentanmeldungen der Anmelderin erstmals beschrieben wurde, neben bereits in der Literatur diskutierten Sepsismarkern als neuartige Sepsismarker u.a. die Proteine "Inflammin" (WO 02/085937), CHP (WO 03/005035), lösliche Cytokeratin-1-Fragmente (sCY1F; WO 03/002600), das Proteine LASP-1 (WO 03/089934) sowie Enzyme wie Aldose-1-Epimerase (Mutarotase; WO 03/048780), Glycin-N-Acyl-Transferase (GNAT; WO 03/048781) und lösliche Carbamoylphosphat Synthetase 1 (CPS 1; WO 03/089933) identifiziert. Eine Diskussion des angewandten Verfahrens der Proteomanalyse und der Ergebnisse, die für einen unter Heranziehung des Verfahrens etablierten Sepsismarker erhalten wurden, der in Form eines midregionalen Fragments des Vorläufers des Hormons ANP (atrial-natriuretisches Peptid) bestimmbar ist, ist veröffentlicht in J. Struck et al., Immuno-analyse & biologie spécialisée 19 (2004) 131-137.

Der Inhalt der genannten älteren Anmeldungen der Anmelderin und der genannten einschlägigen Veröffentlichngen ist durch die ausdrückliche Bezugnahme auf diese Anmeldungen und Veröffentlichungen als ergänzender Teil der Offenbarung der vorliegenden Anmeldung anzusehen.

Die vorliegende Erfindung beruht darauf, dass bei einer Untersuchung der beschriebenen Art unter Verwendung von Leberextrakten von Pavianen eine nur in den Extrakten der mit LPS behandelten Paviane vorkommende, jedoch bei gesunden Pavianen fehlende Substanz isoliert wurde, die - wie im experimentellen Teil näher erläutert wird - als kurzkettige SRL-Alkoholdehydrogenase (SCAD-SRL bzw. DHRS4) identifiziert werden konnte.

Demgemäß betrifft die vorliegende Erfindung gemäß Anspruch 1 im weitesten Sinne die Verwendung von kurzkettiger SRL-Alkoholdehydrogenase (DHRS4) als humoraler Biomarker zum diagnostischen Nachweis und für die Verlaufsprognose sowie die Verlaufs- und Therapiekontrolle von Entzündungen und Infektionen, insbesondere von solchen, die Teil des komplexen Verlaufs eines septischen Krankheitsgeschehens sind.

Die bevorzugte diagnostische Verwendung für die ex vivo Sepsisdiagnostik ist in Anspruch 2 wiedergegeben.

Die Ansprüche 3 bis 10 betreffen bevorzugte Verfahren zur Sepsisdiagnose und deren bevorzugte Ausgestaltungen.

Wie im experimentellen Teil näher beschrieben wird, wurde bei Untersuchungen der Anmelderin eine peptidische Substanz identifiziert, die als kurzkettige SRL-Alkoholdehydrogenase (DHRS4) identifiziert werden konnte

DHRS4 ("dehydrogenase/reductase (SDR family) member 4", Synonyme: SCAD-SRL ("short-chain alcohol dehydrogenase" mit der C-terminalen Sequenz SRL), peroxisomal short-chain alcohol dehydrogenase, u.a.) gehört einer großen Familie von NAD- oder NADP-abhängigen Oxidoreduktasen an (short-chain dehydrogenases/reductases family (SDR)) (1) (http://www.sanger.ac.uk/cgi-bin/Pfam/getacc?PF00106). Diese Enzyme umfassen ca. 250 bis 300 Aminosäuren und liegen meist als Homodi- oder -tetramere vor. Typischerweise enthalten die SDRs zwei Domänen: eine, die das Coenzym (NAD- oder NADP) bindet, die andere, die das Substrat bindet, die Substratspezifität bestimmt und an der Katalyse beteiligt ist. Von der hier interessierenden DHRS4 ist die Substratspezifität experimentell nicht belegt. Aufgrund von Sequenzähnlichkeit wird jedoch vermutet, dass DHRS4 all-trans-Retinal und 9-cis Retinal reduziert. Weitere Substrate könnten Alkylphenylketone und alpha-Dicarbonyl-Verbindungen mit aromatischen Ringen (wie Pyrimidin-4-aldehyd, 3-Benzoylpyridin, 4-Benzoylpyridin, Menadion und 4-Hexanoylpyridin) sein (http://us.expasy.org/,cgi-bin/niceprot.pl?q9nv08).

DHRS4 wird von einem Gen auf Chromosom 14 kodiert (http://genecards.bcgsc.bc.ca/cgibin/carddisp?DHRS4&search=dhrs4&suff=txt). Die Aminosäuresequenz der DHRS4 ist von der zugehörigen cDNA Sequenz abgeleitet worden. In der Literatur liegen unterschiedliche Interpretationen vor, was den N-terminalen Bereich des Proteins betrifft. So geben beispielsweise Clark et al. die 278 Aminosäuren umfassende Sequenz gemäß SEQ ID NO:1 an (2). Andere Autoren sehen den Beginn des Proteins erst beim Methionin in Position 18 der Sequenz gemäß SEQ ID NO:1 (3).

Legt man die längere Variante zugrunde, so kann N-terminal eine Signalsequenz identifiziert werden, welche die Sekretion des Proteins signalisieren könnte (2). Ergebnisse von Fransen et al. legen eher eine peroxisomale Lokalisation nahe, welche durch das C-terminale Tripeptid SRL signalisiert wird. Eine derartige C-terminale Struktur ist als PTS (peroxisomal targeting sequence) bekannt (4).

DHRS4 wird offenbar in diversen Geweben/Organen exprimiert (http://genecards.bcgsc.bc.ca/cgi-bin/carddisp?DHRS4&search=dhrs4&suff=txt).

Zum DHRS4 Gen sind ferner verschiedene alternative Splicing Varianten beschrieben: Vom NCBI werden derzeit 30 unterschiedlich gesplicte Transkripte und entsprechend 30 unterschiedliche Translationsprodukte vorhergesagt (http://www.ncbi.nlm.nih.gov/IEB/Research/Acembly/av.cgi?db= human&c=locusid&1=10901). In der Datenbank SWISSPROT sind allerdings nur zwei zusätzliche Varianten beschrieben.

In der WO0153486 werden mögliche Glykosylierungs- und Myristoylierungsstellen in DHRS4 vorhergesagt, die allerdings nicht experimentell belegt sind.

In der vorliegenden Anmeldung soll die Bezeichnung DHRS4 neben mono- oder multimere Proteine gemäß SEQ ID NO:1 auch Splicingvarianten, sowie Fragmente und anderweitig posttranslational modifizierte Varianten umfassen, insbesodere solche, die in den nachfolgend beschriebenen Assays eine DHRS4 entsprechende Immunreaktivität zeigen.

In pathophysiologischen Zusammenhängen wird in der WO0153486 offenbart, dass u.a. DHRS4 in bestimmten Krebszellen überexprimiert wird und daher als Angriffspunkt für die Therapie von Krebs sowie Diagnose aus Gewebeproben dienen könnte. Ein Zusammenhang mit Infektionen und septischen Krankheitsgeschehen wird dabei jedoch nicht hergestellt.

Das geschieht erstmals in der vorliegenden Anmeldung, die insbesondere die Nutzung der neuen Erkenntnisse für die in *vitro (ex vivo)* Diagnostik betrifft.

In der WO 2004/003162 A2 wird beschrieben, dass in einer cDNA Bibliothek auf der Basis einer RNA aus dem Dünndarm einer fettleibigen weiblichen Person eine cDNA identifiziert wurde, die für ein Peptid aus 244 Aminosäuren kodiert, das zur Familie der kurzkettigen Alkohodehydrogenasen gehört.

In der Anmeldung US 2002/0160392 A1 wird die humane kurzkettige Alkoholdehydrogenase, abgeleitet von einer isolierten und identifizierten DNA aus Hepatoblastomazellen und bezeichnet als PRO1800, in einem Zusammenhang erwähnt, bei dem es vorrangig um eine potentielle Arzneimittelentwicklung geht.

Die Bestimmung von DHRS4 zu diagnostischen Zwecken in den biologischen Flüssigkeiten erfolgt vorzugsweise ex vivo mit Hilfe immundiagnostischer Bestimmungsverfahren (Ligandenbindungsassays; Immunoassays).

Es versteht sich dabei, dass, die erforderliche Spezifität und Empfindlichkeit vorausgesetzt, irgendwelche nach bekannten Prinzipien arbeitenden Ligandenbindungsassays/Immunoassays zur quantitativen oder semiquantitativen Bestimmung von DHRS4 in biologischen Flüssigkeiten, insbesondere solchen aus der Zirkulation wie Serum oder Plasma, eingesetzt werden können.

Bei einer bevorzugten Ausführungsform wird das Verfahren als heterogener Sandwich-Immunoassay durchgeführt, bei dem ein erster DHRS4 bindender Antikörper an eine beliebige Festphase, beispielsweise die Wände beschichteter Teströhrchen (z.B. aus Polystyrol; "Coated Tubes"; CT) oder an Mikrotiterplatten, zum Beispiel aus Polystyrol, oder an Partikel, beispielsweise Magnetpartikel immobilisiert ist, während ein weiterer für DHRS4 spezifischer Antikörper einen Rest trägt, der ein direkt nachweisbares Label darstellt oder eine selektive Verknüpfung mit einem Label ermöglicht und der Detektion der gebildeten Sandwich-Strukturen dient. Auch eine zeitlich verzögerte bzw. nachträgliche Immobilisierung unter Verwendung geeigneter Festphasen ist möglich.

Grundsätzlich können alle in Assays der beschriebenen Art verwendbaren Markierungstechniken angewandt werden, zu denen Markierungen mit Radioisotopen, Enzymen, Fluoreszenz-, Chemolumineszenz- oder Biolumineszenz-Labeln und direkt optisch detektierbaren Farbmarkierungen, wie beispielsweise Goldatomen und Farbstoffteilchen, wie sie insbesondere für sog. Point-of-Care (POC) oder Schnelltests verwendet werden, gehören. Es liegt somit im Rahmen der vorliegenden Erfindung, das erfindungsgemäße Verfahren auch als Schnelltest auszugestalten.

Das Verfahren zur Bestimmung von DHRS4 kann außerdem beispielsweise auch unter Anwendung eines homogenen Nachweisverfahrens durchgeführt werden, bei dem aus zwei Antikörpern und dem nachzuweisenden DHRS4 gebildete Sandwichkomplexe in der flüssigen Phase suspendiert bleiben. In einem solchen Fall ist es bevorzugt, beide Antikörper mit Teilen eines Nachweissystems zu markieren, das dann, wenn beide Antikörper in einen einzigen Sandwich integriert werden, eine Signalerzeugung oder Signalauslösung ermöglicht. Derartige Techniken sind insbesondere als Fluoreszenzverstärkungs- oder Fluoreszenzlöschungs-Nachweisverfahren ausgestaltbar. Ein besonderes bevorzugtes derartiges Verfahren betrifft die Verwendung von paarweise einzusetzenden Nachweisreagenzien, wie sie beispielsweise beschrieben sind in US-A-4 822 733, EP-B1-180 492 oder EP-B1-539 477 und dem darin zitierten Stand der Technik. Sie ermöglichen eine Messung, die selektiv nur Reaktionsprodukte erfaßt, die beide Markierungskomponenten in einem einzigen Immunkomplex enthalten, direkt in der Reaktionsmischung. Als Beispiel ist auf die unter den Marken TPACE^{®} (Time Resolved Amplified Cryptate Emission) bzw. KRYPTOR^{®} angebotene Technologie zu verweisen, die die Lehren der o.g. Anmeldungen umsetzt.

Zwei für DHRS4 spezifische Antikörper können aber auch im Falle heterogener Sandwich-Immunoassays Teile eines Nachweissystems der soeben im Zusammenhang mit homogenen Assays beschriebenen Art aufweisen.

Wie nachfolgend gezeigt wird, ist das Auftreten - i.S. einer nachweisbaren Expression - von DHRS4 in Pavian-Leberextrakten an einen vorausgehenden "Sepsis"-auslösenden Endotoxin-Reiz bzw. eine vorausgehende Infektion gekoppelt. Bei unbehandelten Kontrollen ist, im Einklang mit der eingangs zitierten Literatur, kein DHRS4-Nachweis möglich. Damit stellt DHRS4 einen potentiellen Sepsismarker dar.

Nachfolgend wird die Erfindung unter Bezugnahme auf sieben Figuren noch näher erläutert.

In den Figuren zeigen:

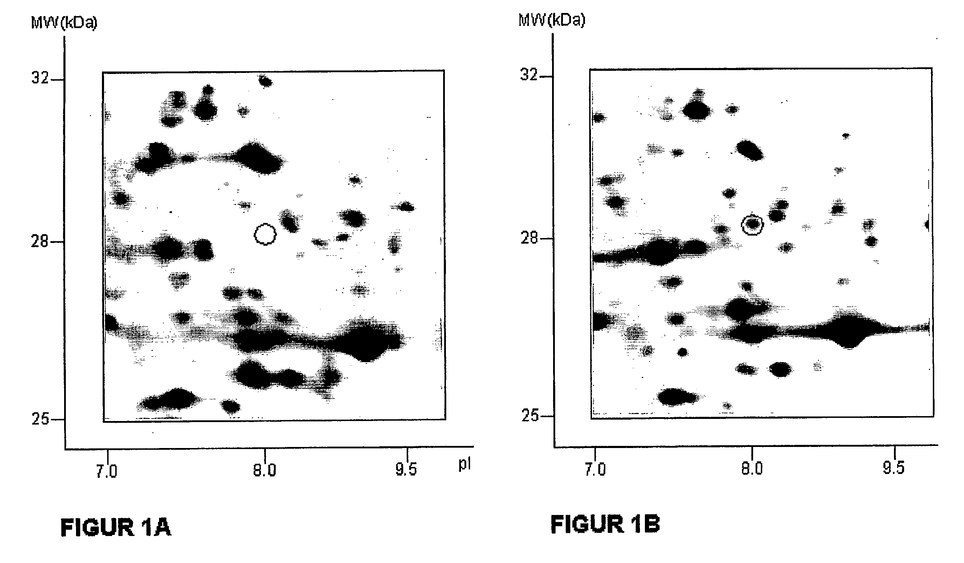

- Fig. 1:: vergrösserte Ausschnitte von 2D-Gelen, die einen Vergleich der Spotmuster von löslichen Leberpro- teinen eines gesunden Pavians (A) mit den Leber- proteinen eines Pavians 5 h nach einer durch In- jektion von LPS induzierten Sepsis (B) ermögli- chen. Der Kreis zeigt die Position des erfindungs- gemässen sepsisspezifischen Produkts DHRS4 an.

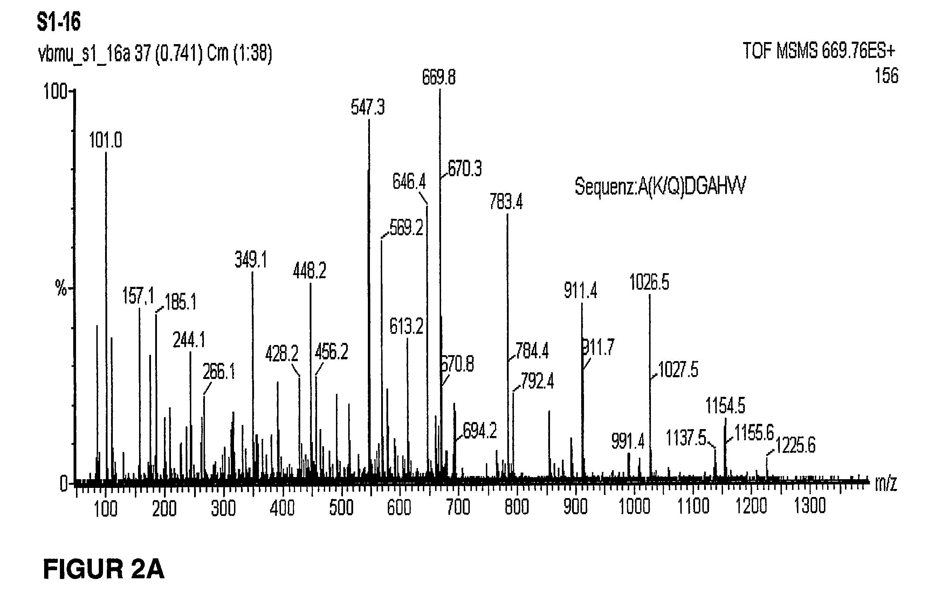

- Fig. 2A:: Ergebnisse einer ESI-MS/MS Tandem-Massenspektrome- trie eines selektierten Peptidfragments der Tryp- sinverdauung des sepsisspezifischen Proteinspots. Tandem-Mass-Spektrum des Precursorions = 669.76. Die Interpretation des Spektrums resultiert in der Aminosäuresequenz A(K/Q)DGAHVV.

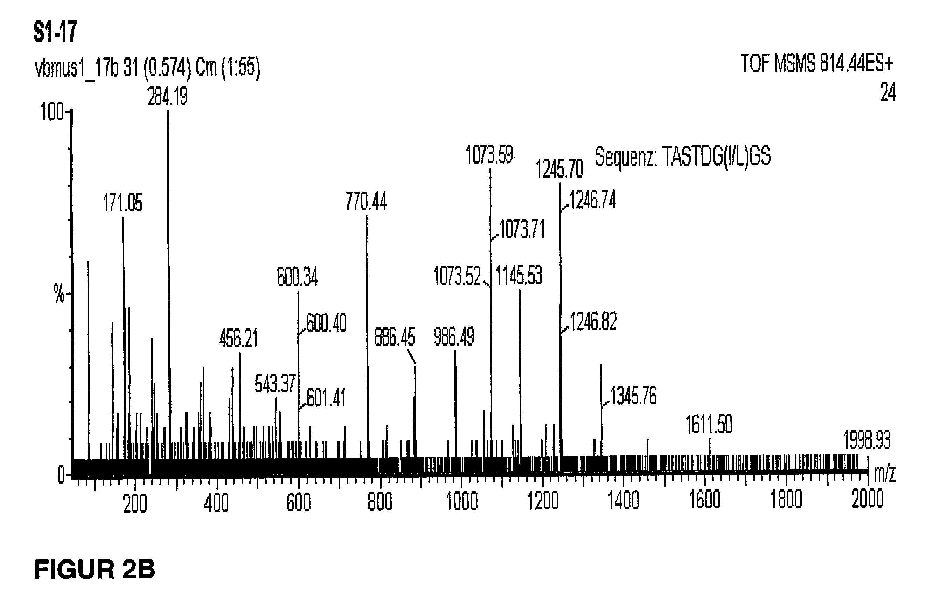

- Fig. 2B:: Ergebnisse einer ESI-MS/MS Tandem-Massenspektrome- trie eines selektierten Peptidfragments der Tryp- sinverdauung des sepsisspezifischen Proteinspots. Tandem-Mass-Spektrum des Precursorions = 814.44. Die Interpretation des Spektrums resultiert in der Aminosäuresequenz TASTDG(I/L)GS.

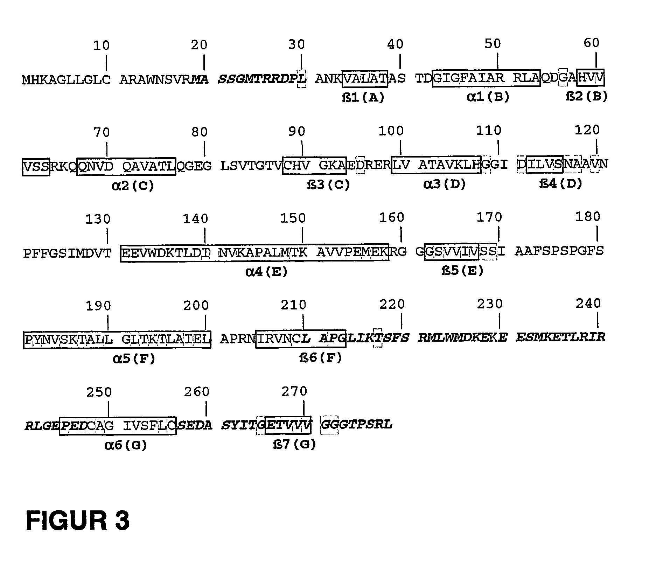

- Fig. 3:: die Aminosäuresequenz von DHRS4 (vgl. auch SEQ ID NO:1). Interessierende Strukturelemente sind her- vorgehoben: Wahrscheinliche β-Faltblattstrukturen und α-Helices wurden durch Vergleich mit den in Kallberg Y et al., (2002) (15) und in Jörnvall H et al., (1995) (1) angegebenen Sequenzen identifi- ziert und sind eingerahmt. Durch denselben Ver- gleich wurden konservierte Aminosäuren identifi- ziert; diese sind schattiert unterlegt. Für andere Mitglieder der SDR-Familie wurde beschrieben, dass Kontaktstellen bei einer Multimerisierung die Bereiche α4(E) und α5(F) sind; Jörnvall H et al.; (1995) (1). Bindungsstelle für NAD/NADP ist nach Sequenzvergleich mit DHS2 (SWISSPROT Eintrag Q13268) der Bereich Pos. 36-59. Sequenzbereiche, die als Peptide zur Immunisierung benutzt wurden, sind fett und kursiv angegeben (Pos. 19-30, 209-228, 230-247, 256-278).

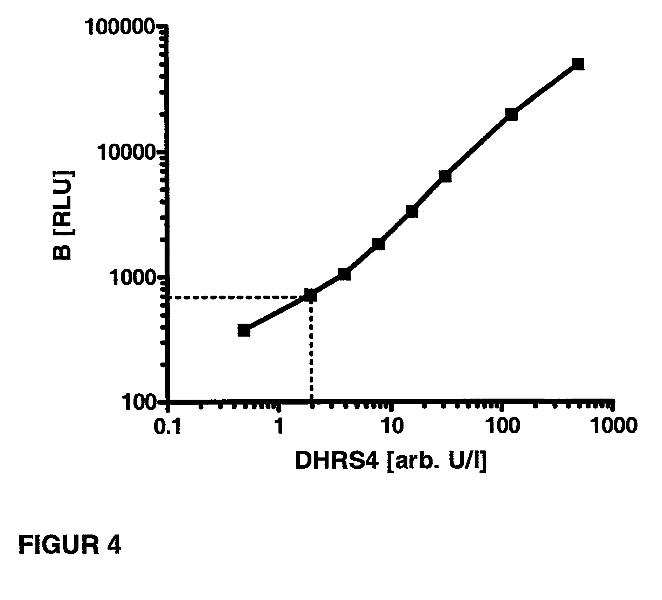

- Fig. 4:: eine typische Standardkurve des in der vorliegen- den Anmeldung in Kapitel 5.1 für Bestimmungen verwendeten Sandwichimmunoassays für DHRS4 (Chemi- lumineszenz-Assay im Coated Tube Format; Antikör- per gegen die Bereiche Pos. 209-228 (Peptid PLE 20; Festphase) und 256-278 (Peptid PSL23; Tracer). Als Standards dienten Verdünnungen von rekombinan- te DHRS4 enthaltendem E. coli-Extrakt in Pferden- ormalserum, denen arbiträre Einheiten zugeschrie- ben wurden. Die funktionale Assaysensitivität von 2 U/l ist angezeigt.

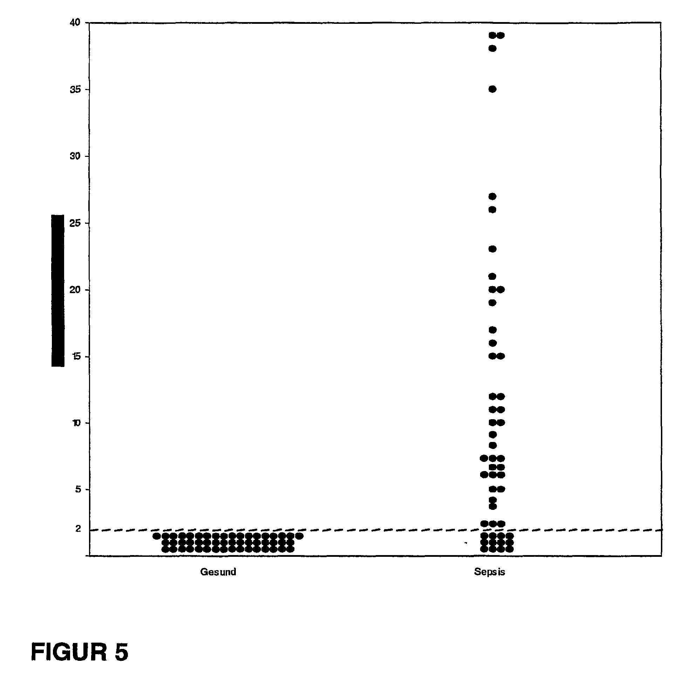

- Fig. 5:: mit einem Assay gemäß Fig. 4 gemessene DHRS4 Im- munreaktivitäten, gemessen in je 50 Plasmaproben von gesunden Probanden sowie Sepsis-Patienten. Die funktionale Assaysensitivität bei 2 U/l ist ange- zeigt.

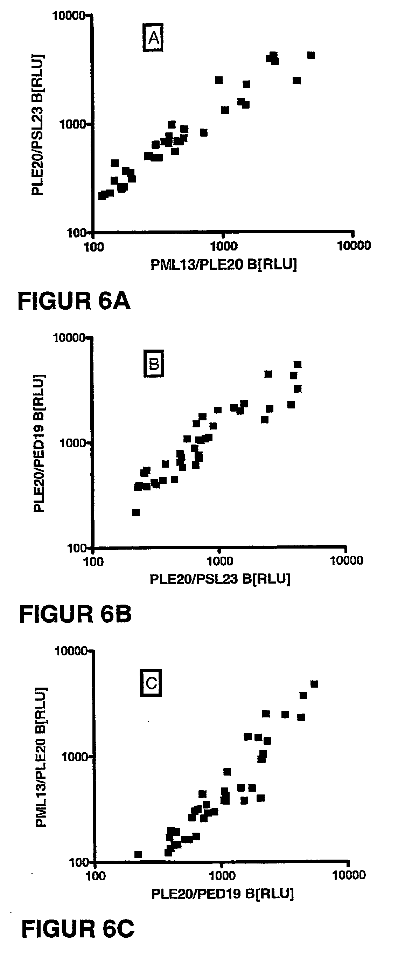

- Fig. 6:: einen Methodenvergleich verschiedener Sandwichas- says für DHRS4: Plasmaproben wurden in verschiede- nen Assays vermessen. Die Peptid-Epitope der in den Assays verwendeten Antikörper sind an den Achsen angegeben. Die Korrelationskoeffizienten betragen: r=0.97 (A), r=0.95 (B), r=0.94 (C).

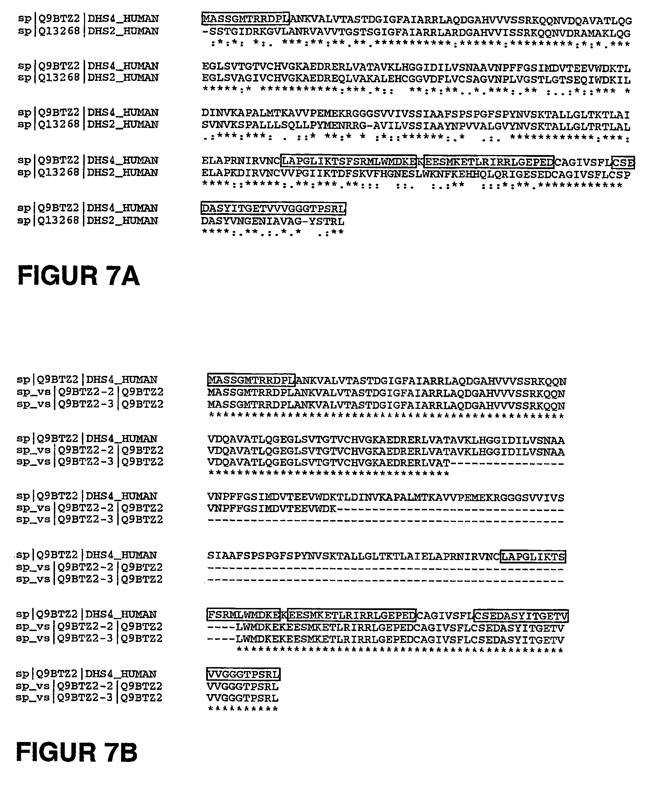

- Fig. 7A:: einen Sequenzvergleich von DHRS4 (DHS4_HUMAN) mit der Sequenz DHS2_HUMAN (Programm ClustalW). Iden- tische Aminosäuren sind mit Sternchen gekennzeich- net; strukturell verwandte Aminosäuren mit Punk- ten. Markiert sind solche Sequenzbereiche von DHRS4, die als Peptide synthetisiert und für die Erzeugung von Antikörpern verwendet wurden.
- Fig. 7B:: einen Sequenzvergleich von DHRS4 (DHS4_HUMAN) mit zwei in der Datenbank SWISSPROT identifizierten Splicing-Varianten (Programm ClustalW). Identische Aminosäuren sind mit Sternchen gekennzeichnet. Markiert sind solche Sequenzbereiche von DHRS4, die als Peptide synthetisiert und für die Erzeu- gung von Antikörpern verwendet wurden.

### Versuchsergebnisse:

### 1. Infektionssimulation durch Endotoxinverabreichung im Tiermodell (Paviane)

In Anlehnung an die mit Pavianen durchgeführten Versuche zur Stimulierung der Procalcitonin-Ausschüttung durch Endotoxininjektionen (5, 6) wurden männlichen Pavianen (Papio ursinus), ca. 2 Jahre alt, 27 bis 29 kg schwer) jeweils 100 µg LPS (Lipopolysaccharid aus Salmonella typhimurium, Bezugsquelle: Sigma) pro kg Körpergewicht intravenös verabreicht. 5 bis 5,5 h nach der Injektion wurden die Tiere durch intravenöse Verabreichung von 10 ml Doletal getötet. Unmittelbar nach ihrem Exitus wurde die Leber präpariert und durch Einfrieren in flüssigem Stickstoff stabilisiert.

Bei der weiteren Verarbeitung wurden Proben der tiefgefrorenen Leber (1 g) unter Stickstoffkühlung mit 1.5 ml Puffer A (50 mM Tris/HCl, pH 7.1, 100 mM KCl, 20% Glycerol, 1 mM Pefabloc, Protease Inhibitor Cocktail "Complete" (Roche): pro 1.5 ml Puffer A: 0.2 µl einer Lösung von 1 Tablette in 2 ml) versetzt und in einem Porzellanmörser zu einem Mehl pulverisiert (7). Das Homogenat wurde 6 x 10 sec in einem Wasserbad mit Ultraschall behandelt, zentrifugiert für 40 min bei 100.000 g, 4°C, und der erhaltene Überstand wurde gewonnen. Das verbleibende Zellpellet wurde wie oben beschrieben ein weiteres mal unter Stickstoff pulverisiert, ultraschallbehandelt und zentrifugiert. Der daraus resultierende zweite Überstand wurde mit dem ersten Überstand vereinigt und bis zur weiteren Verarbeitung bei -80° C gelagert.

### 2. Vergleichende Proteomanalyse unter Verwendung von Leberextrakten von Pavianen

Leberproteinextrakte von einerseits gesunden Pavianen (Kontrolle) und andererseits Pavianen, denen LPS injiziert worden war, wurden für eine Proteomanalyse verwendet. Bei der analytischen 2D-Gelelektrophorese wurde Leberextrakt, 150 µg Protein enthaltend, auf 9 M Harnstoff, 70 mM DTT, 2% Ampholyte pH 2-4 eingestellt und dann mittels analytischer 2D-Gelelektrophorese aufgetrennt (8). Die Sichtbarmachung der Proteine im 2D-Elektrophoresegel erfolgte mittels Silberfärbung (9).

Zur Auswertung wurden die Proteinspotmuster der Proben unbehandelter Tiere mit den Proteinspotmustern verglichen, die aus Lebergewebeproben behandelter Tiere resultierten. Fig. 1 zeigt einen Vergleich der 2D-Elektrophoresegele für eine Kontrollprobe (A) und eine Probe eines mit LPS behandelten Tieres (B). Der zusätzliche Proteinspot in (B) mit einem apparenten Molekulargewicht von ca. 29000 Dalton und einem apparenten isoelektrischen Punkt von ca. 8.0 bis 8.2 wurde massenspektrometrisch analysiert, nachdem das im Proteinspotmuster der analytischen 2D-Gelelektrophorese identifizierte differentiell auftretende Protein anschließend noch mittels präparativer 2D-Gelelektrophorese unter Einsatz von 350 µg Protein präpariert worden war.

Bei der präparativen 2D-Gelelektrophorese erfolgte die Färbung mittels Coomassie Brilliant Blue G250 (10).

Der für die weitere Analyse vorselektierte Proteinspot wurde aus dem Gel ausgeschnitten und unter Anwendung einer publizierten Methode trypsinverdaut (11) und anschließend die generierten Trypsinfragmente mittels Elektrospray Massenspektrometrie (ESI-MS) analysiert (12-14). Es wurde ein Q-TOF-Massenspektrometer mit einer nanoflow-Z-Spray-Ionenquelle der Firma Micromass, UK, verwendet. Dabei wurde entsprechend der Arbeitsanleitung des Geräteherstellers gearbeitet.

### 3. Identifizierung von kurzkettiger SRL-Alkoholdehydrogenase (DHRS4)

Aus dem Mutterspektrum des mit Trypsin verdauten Proteins des selektierten Spots wurden jeweils einzelne Peptide ("Tags") durch ESI-MS/MS identifiziert. Die für diese Fragmente erhaltenen Massenspektren konnten auf an sich bekannte Weise rechnerisch ausgewertet werden. Die Datenbanksuche mit den ESI-MS/MS Sequenztags wurde mit Hilfe von MS-Edman (http://falcon.ludwig.ucl.ac.uk/ucsfhtml3.2/msedman.htm) durchgeführt, welche auf dem Server Protein Prospector der University of California San Francisco Mass Spectrometry Facility lokalisiert ist.

Fig. 2A zeigt exemplarisch das MS/MS-Ergebnis für das Peptid mit der Masse 669.76 und der aus dem Spektrum herauszulesenden Sequenz A(K/Q)DGAHVV (K und Q können massenspektrometrisch nicht unterschieden werden). Fig. 2B zeigt ein weiteres Peptid mit der Masse von 814.44 und das daraus resultierende Spektrum und Aminosäuresequenz von TASTDG(I/L)GS (I und L können massenspektrometrisch nicht unterschieden werden).

Beide Aminosäuresequenzen wurden mit allen Sequenzen der Säugetierproteine der NCBInr.18.09.00 bzw. NCBInr. 6.17.2000 Datenbank verglichen. Die beste Übereinstimmung für beide Peptide (A(K/Q)DGAHVV und TASTDG(I/L)GS) wurde mit Teilen des humanen Proteins DHRS4 (peroxisomal short chain alcohol dehydrogenase) gefunden: AQDGAHVV und TASTDGIGF. Diese Identifizierung ist als zweifelsfrei anzusehen. Für die humane DHRS4 (SEQ ID NO:1) lässt sich ein Molekulargewicht von 29.5 kDa und ein isoelektrischer Punkt von 8.8 berechnen. Diese Werte liegen sehr nahe an den experimentell beobachteten Werten (s.o.) und unterstützen das Identifizierungsergebnis.

Aufgrund der hohen Ähnlichkeit der pathophysiologischen Reaktionen von Pavianen und Menschen, die sich auch gerade in den zahlreichen oben diskutierten Untersuchungen der Anmelderin immer wieder gezeigt hat, die auf den Ergebnissen einer künstlich induzierten Sepsis bei Pavianen aufbauen, kann davon ausgegangen werden, dass bei infizierten bzw. septischen menschlichen Patienten im wesentlichen identische Verhältnisse auftreten wie im beschriebenen Pavian-Tiermodell. Durch immundiagnostische Bestimmung von DHRS4 in humanen Plasmen von Gesunden und Sepsispatienten bestätigte sich diese Grundannahme.

Zu diesem Zweck wurden Sandwichimmunoassays für DHRS4 entwickelt und damit das Vorkommen von DHRS4 in der Blutzirkulation verschiedener Patientengruppen untersucht.

### 4. Herstellung von Assavkomponenten für DHRS4 Immunoassays

### 4.1. Peptidsynthesen

Zur Erzeugung von Antikörpern gegen DHRS4 wurden Teilpeptide aus der Aminosäuresequenz von DHRS4 synthetisiert, mit denen Schafe immunisiert wurden (Fig. 3). Bei der Auswahl der Peptide wurden Informationen zur Sekundär-, Tertiär- und Quartärstruktur des Proteins berücksichtigt, mit dem Ziel, Antikörper zu erzeugen, die geeignet sind, natives DHRS4 zu binden.

Abgeleitet von der bekannten Aminosäuresequenz von DHRS4 wurden vier Bereiche ausgewählt (Pos. 19-30, 209-228, 230-247, 256-278). Die Bereiche wurden (Pos. 19-30 und 230-247 jeweils ergänzt um einen N-terminalen Cystein-Rest) nach Standardverfahren als lösliche Peptide chemisch synthetisiert, gereinigt, mittels Massenspektrometrie und Reversed Phase HPLC qualitätskontrolliert und in Aliquots lyophilisiert (Firma JERINI AG, Berlin, Deutschland). Die Aminosäuresequenzen der Peptide lauten:

| | |
|---|---|
| PML13 | CMASSGMTRRDPL (SEQ ID NO:2) |
| PLE20 | CLAPGLIKTSFSRMLWMDKE (SEQ ID NO:3) |
| PED19 | CEESMKETLRIRRLGEPED (SEQ ID NO:4) |
| PSL23 | CSEDASYITGETVVVGGGTPSRL (SEQ ID NO:5) |

### 4.2. Konjugation und Immunisierung

Mittels MBS (m-Maleimidobenzoyl-N-hydroxysuccinimid Ester) wurden die Peptide PML13, PLE20, PED19 und PSL23 an das Trägerprotein KLH (Keyhole limpet hemocyanin) konjugiert (s. Arbeitsanleitung "NHS-Esters-Maleimide Crosslinkers" Firma PIERCE, Rockford, IL, USA). Mit diesen Konjugaten wurden Schafe nach folgendem Schema immunisiert: Jedes Schaf erhielt initial 100 µg Konjugat (Massenangabe bezogen auf den Peptid-Anteil des Konjugats) und anschließend 4-wöchentlich je 50 µg Konjugat (Massenangabe bezogen auf den Peptid-Anteil des Konjugats). Beginnend mit dem vierten Monat nach Beginn der Immunisierung wurden 4-wöchentlich je Schaf 700 ml Blut abgenommen und daraus durch Zentrifugation Antiserum gewonnen. Konjugationen, Immunisierungen und Gewinnung von Antiseren wurden von der Firma MicroPharm, Carmarthenshire, UK, durchgeführt.

### 4.3. Reinigung der Antikörper

In einem 1-Schritt Verfahren wurden aus den Antiseren, die beginnend mit dem vierten Monat nach der Immuniserung gewonnen worden waren, die Peptid-spezifischen Antikörper präpariert.

Dazu wurden zunächst die Peptide PML13, PLE20, PED19 und PSL23 an SulfoLink Gel gekoppelt (s. Arbeitsanleitung "SulfoLink Kit" Firma PIERCE, Rockford, IL, USA). Dabei wurden zur Kopplung je 5 mg Peptid pro 5 ml Gel angeboten.

Die Affinitätsreinigung von Peptid-spezifischen Antikörpern aus Schaf Antiseren gegen die Peptide wurde wie folgt durchgeführt:

Die Peptid-Säulen wurden zunächst drei mal im Wechsel mit je 10 ml Elutionspuffer (50 mM Citronensäure, pH 2.2) und Bindungspuffer (100 mM Natriumphosphat, 0.1% Tween, pH 6.8) gewaschen. 100 ml der Antiseren wurden über 0,2 µm filtriert und mit dem vorhandenen Säulenmaterial versetzt. Dazu wurde das Gel quantitativ mit 10 ml Bindungspuffer aus der Säule gespült. Die Inkubation erfolgte über Nacht bei Raumtemperatur unter Schwenken. Die Ansätze wurden quantitativ in Leersäulen (NAP 25, Pharmacia, entleert) überführt. Die Durchläufe wurden verworfen. Anschließend wurde mit 250 ml Bindungspuffer proteinfrei (Proteingehalt des Wascheluats <0,02 A 280 nm) gewaschen. Auf die gewaschene Säulen wurde Elutionspuffer gegeben, und es wurden Fraktionen à 1 ml gesammelt. Von jeder Fraktion wurde der Proteingehalt mittels der BCA-Methode (s. Arbeitsanleitung Firma PIERCE, Rockford, IL, USA) bestimmt. Fraktionen mit Proteinkonzentrationen > 0.8 mg/ml wurden gepoolt. Nach Proteinbestimmung der Pools mittels der BCA-Methode ergaben sich Ausbeuten von 57 mg für den anti-PML13 Antikörper, 56 mg für den anti-PLE20 Antikörper, 12 mg für den anti-PED19 Antikörper und 99 mg für den anti-PSL23 Antikörper.

### 4.4. Antikörper-Markierung

Über NAP-5 Gelfiltrationssäulen (Pharmacia) wurden je 500 µl der gereinigten anti-PLE20-, anti-PED19- und anti-PSL23 Antikörper (s.o.) in je 1 ml 100 mM Kalium-Phosphatpuffer (pH 8,0) nach Arbeitsanleitung umgepuffert. Die Proteinkonzentationen der Antikörperlösungen wurden mit 100 mM Kalium-Phosphatpuffer (pH 8,0) auf 1,5 mg/ml eingestellt.

Zur Chemilumineszenzmarkierung wurden alle Antikörper wie folgt weiterbehandelt: 67 µl der Antikörperlösung wurden mit 10 µl MA70-Akridinium-NHS-Ester (1 mg/ml; Firma HOECHST Behring) versetzt und 15 Minuten bei Raumtemperatur inkubiert. Dann wurden 423 µl 1 M Glycin zugesetzt und weitere 10 Minuten inkubiert. Anschließend wurde der Markierungsansatz über eine NAP-5 Gelfiltrationssäule (Pharmacia) in 1 ml Laufmittel A (50 mM Kaliumphosphat, 100 mM NaCl, pH 7.4) nach Arbeitsanleitung umgepuffert und dabei von niedermolekularen Bestandteilen befreit. Zur Abtrennung letzter Reste nicht an Antikörper gebundenen Labels wurde eine Gelfiltrations-HPLC durchgeführt (Säule: Waters Protein Pak SW300). Die Probe wurde aufgetragen und bei einer Flußrate von 1 ml/min mit Laufmittel A chromatographiert. Mit einem Duchflußphotometer wurden die Wellenlängen 280 nm und 368 nm gemessen. Das Absorptionsverhältnis 368 nm/280 nm als Maß für den Markierungsgrad des Antikörpers betrug am Peak 0.10 +/- 0.01. Die monomeren Antikörper enthaltenden Fraktionen (Retentionszeit 8-10 min) wurden gesammelt und in 3 ml 100 mM Natriumphosphat, 150 mM NaCl, 5% Bovines Serum Albumin, 0.1% Natrium Azid, pH 7.4, gesammelt.

### 4.5. Antikörper-Immobilisierung

Bestrahlte 5 ml Polystyrolröhrchen (Firma Greiner) wurden mit gereinigtem anti-PML13 Antikörper bzw. anti-PLE20 Antikörper wie folgt beschichtet: Der Antikörper wurde in 50 mM Tris, 100 mM NaCl, pH 7.8 zu einer Konzentration von 6.6 µg/ml verdünnt. In jedes Röhrchen wurden 300 µl dieser Lösung pipettiert. Die Röhrchen wurden 20 Stunden bei 22°C inkubiert. Die Lösung wurde abgesaugt. Dann wurde jedes Röhrchen mit 4.2 ml 10 mM Natriumphosphat, 2% Karion FP, 0.3% Bovines Serum Albumin, pH 6.5 befüllt. Nach 20 Stunden wurde die Lösung abgesaugt. Schließlich wurden die Röhrchen in einem Vakuumtrockner getrocknet.

### 5. Durchführung und Auswertung von DHRS4 Immunoassays

### 5.1. Bestimmung von DHRS4 in humanen Plasmen

Es wurde ein zuerst Sandwichimmunoassay für DHRS4 (Antikörper gegen die Bereiche Pos. 209-228 Bzw. Peptid PLE20 sowie 256-278 bzw. Peptid PSL23) mit einer funktionalen Assaysensitivität von ca. 2 U/l entwickelt (typische Standardkurve: vgl. Fig. 4).

Mit diesem Assay wurden Plasmaproben von Gesunden und Sepsispatienten vermessen (Fig. 5). Sämtliche Proben von Gesunden ergaben Messwerte unterhalb der Funktionalen Assaysensitivität; 38 der 50 Proben von Sepsispatienten ergaben Messwerte oberhalb der Funktionalen Assaysensitivität. Es ergibt sich eine diagnostische Sensitivität von 76% bei 100% Spezifität.

### 5.2. Immundiagnostische Bestimmung von DHRS4 in humanen Seren - Methodenvergleich

In einem Methodenvergleich wurde gezeigt, dass Sandwichassays, die Antikörper gegen andere Epitope der DHRS4 verwenden, zu vergleichbaren Ergebnissen wie der unter 5.1 eingesetzte Assay führen (Fig. 6).

Dazu wurden insgesamt drei Sandwichimmunoassays für DHRS4 etabliert, die mit folgenden Antikörpern arbeiteten:
a) Festphase: anti-PLE20, Tracer: anti-PSL23 (vgl. 5.1.)
b) Festphase: anti-PLE20, Tracer: anti-PED19
c) Festphase: anti-PML13, Tracer: anti-PLE20

Als Standardmaterial diente jeweils rekombinante humane DHRS4 in Form eines Extrakts aus transformierten E. coli Bakterien (InVivo GmbH, Hennigsdorf, Deutschland). Klonierung und Expression des DHRS4 Gens erfolgten nach molekularbiologischen Standardverfahren. Der Extrakt wurde seriell in Pferdenormalserum (Firma SIGMA) verdünnt. Den so hergestellten Standards wurden Konzentrationen in arbiträren Einheiten zugeschrieben.

Meßproben waren EDTA-Plasmen von augenscheinlich Gesunden und von Patienten mit Sepsis.

Die drei Sandwichimmunoassays wurden in gleicher Weise wie folgt angesetzt: In die jeweiligen mit Antikörpern beschichteten Teströhrchen wurden 100 µl Standards bzw. Proben sowie 100 µl Assaypuffer (100 mM Natriumphosphat, 150 mM NaCl, 5% Bovines Serum Albumin, 0.1% unspez. Schaf IgG, 0.1% Natrium Azid, pH 7.4), enthaltend 1 Million RLU (relative light units) des jeweiligen MA70-markierten Antikörpers, pipettiert. Es wurde 20 Stunden bei 22°C unter Schütteln inkubiert. Dann wurde 4 x mit je 1 ml Waschlösung (0.1% Tween 20) pro Röhrchen gewaschen, abtropfen gelassen und die am Röhrchen gebundene Chemilumineszenz in einem Luminometer (Firma BERTHOLD, LB952T; Basisreagenzien BRAHMS AG) vermessen.

Unter Verwendung der Software MultiCalc (Spline Fit) wurde die DHRS4 Konzentrationen der Proben an der Standardkurve abgelesen.

### 5.3. Abschätzung von möglichen Störeinflüssen für die immundiagnostische DHRS4 Bestimmung

Um abzuschätzen, ob Antikörper, die gegen die ausgewählten Peptide erzeugt wurden, auch mit anderen humanen Proteinen/- Peptiden als DHRS4 (SCAD-SLR) kreuzreagieren könnten, wurde DHRS4 mit allen in der SWISSPROT-Datenbank enthaltenen humanen Sequenzen mittels "Quickblast" verglichen. Näher analysiert wurden solche gefundenen Sequenzen, die Ähnlichkeit mit DHRS4 aufwiesen, und die Bereiche enthielten, welche Epitopen für solche Antikörper entsprechen, die in den durchgeführten drei Sandwichassayvarianten zur Anwendung kamen. Gefunden wurden zwei Splicing-Varianten von DHRS4, sowie das Produkt des DHRS2 Gens (Fig. 7A, 7B). Kreuzreaktivität mit den beiden Splicing Varianten wäre zu erwarten, sofern der anti-PLE20-Antikörper auch nur die Sequenz LWMDKE erkennen würde (was experimentell nicht untersucht wurde). Eine Kreuzreaktivität mit dem Produkt des DHRS2 Gens erscheint jedoch aufgrund der geringen Sequenzhomologie eher unwahrscheinlich, kann aber nicht völlig ausgeschlossen werden.

Da nicht bekannt ist, ob und in welchen Konzentrationen DHRS4 auch in Blutzellen vorliegt, dies aber für die Probennahme relevant ist (Hämolyse, unvollständige Abtrennung von Thrombozyten), wurde EDTA-Blut von normalen Probanden durch Ultraschall vollständig hämolysiert und dann bzgl. DHRS4 vermessen. Dabei wurden Konzentrationen von 1-3 U/l detektiert (Daten nicht gezeigt). Dies zeigt, dass DHRS4 in Blutzellen vorliegt, jedoch in so geringen Konzentrationen, dass partielle Hämolyse oder partielle unvollständige Abtrennung von Thrombozyten keinen relevanten Einfluss auf Messergebnisse haben.

Offen ist, durch welchen Mechanismus DHRS4 bei Sepsis in die Blutzirkulation gelangt.
1. Jornvall, H., Persson, B., Krook, M., Atrian, S., Gonzalez-Duarte, R., Jeffery, J., and Ghosh, D. 1995. Short-chain dehydrogenases/reductases (SDR). Biochemistry 34:6003-6013.
2. Clark, H.F., Gurney, A.L., Abaya, E., Baker, K., Baldwin, D., Brush, J., Chen, J., Chow, B., Chui, C., Crowley, C., et al. 2003. The secreted protein discovery initiative (SPDI), a large-scale effort to identify novel human secreted and transmembrane proteins: a bioinformatics assessment. Genome Res 13:2265-2270.
3. Fransen, M., Van Veldhoven, P.P., and Subramani, S. 1999. Identification of peroxisomal proteins by using M13 phage protein VI phage display: molecular evidence that mammalian peroxisomes contain a 2,4-dienoyl-CoA reductase. Biochem J 340 ( Pt 2):561-568.
4. Gould, S.J., Keller, G.A., Hosken, N., Wilkinson, J., and Subramani, S. 1989. A conserved tripeptide sorts proteins to peroxisomes. J Cell Biol 108:1657-1664.
5. Redl, H., Schlag, G., Togel, E., Assicot, M., and Bohuon, C. 2000. Procalcitonin release patterns in a baboon model of trauma and sepsis: relationship to cytokines and neopterin. Crit Care Med 28:3659-3663.
6. Redl, H., and Schlag, G. 1998. Non-human primate models of sepsis. Sepsis 2:243-253.
7. Klose, J. 1999. Fractionated extraction of total tissue proteins from mouse and human for 2-D electrophoresis. Methods Mol Biol 112:67-85.
8. Klose, J., and Kobalz, U. 1995. Two-dimensional electrophoresis of proteins: an updated protocol and implications for a functional analysis of the genome. Electrophoresis 16:1034-1059.
9. Heukeshoven, J., and Dernick, R. 1988. Improved silver staining procedure for fast staining in PhastSystem Development Unit. I. Staining of sodium dodecyl sulfate gels. Electrophoresis 9:28-32.
10. Neuhoff, V., Arold, N., Taube, D., and Ehrhardt, W. 1988. Improved staining of proteins in polyacrylamide gels including isoelectric focusing gels with clear background at nanogram sensitivity using Coomassie Brilliant Blue G-250 and R-250. Electrophoresis 9:255-262.
11. Otto, A., Thiede, B., Muller, E.C., Scheler, C., Wittmann-Liebold, B., and Jungblut, P. 1996. Identification of human myocardial proteins separated by two-dimensional electrophoresis using an effective sample preparation for mass spectrometry. Electrophoresis 17:1643-1650.
12. Neubauer, G., King, A., Rappsilber, J., Calvio, C., Watson, M., Ajuh, P., Sleeman, J., Lamond, A., and Mann, M. 1998. Mass spectrometry and EST-database searching allows characterization of the multi-protein spliceosome complex. Nat Genet 20:46-50.
13. Lingner, J., Hughes, T.R., Shevchenko, A., Mann, M., Lundblad, V., and Cech, T.R. 1997. Reverse transcriptase motifs in the catalytic subunit of telomerase. Science 276:561-567.
14. Mann, M., and Pandey, A. 2001. Use of mass spectrometry-derived data to annotate nucleotide and protein sequence databases. Trends Biochem Sci 26:54-61.
15. Kallberg, Y., Oppermann U., Jörnvall H. and Persson, B., Eur. J. Biochem. 269, 4409-4417 (2002)

## Patentansprüche

1. Verwendung von kurzkettiger SRL-Alkoholdehydrogenase (DHRS4) mit der Aminosäuresequenz gemäß SEQ ID NO:1 als humoraler Biomarker zum diagnostischen Nachweis und für die Verlaufsprognose sowie die Verlaufs- und Therapiekontrolle von Entzündungen und Infektionen.

2. Verwendung nach Anspruch 1 im Rahmen der differentialdiagnostischen Früherkennung und Erkennung, zur Bestimmung des Schweregrads und für die Verlaufs- und Therapiekontrolle von Sepsis und schweren Infektionen durch *ex vivo* Bestimmung des Auftretens und/oder der Menge von DHRS4 mit der Aminosäuresequenz gemäß SEQ ID NO:1 und/oder von pathophysiologisch auftretenden Fragmenten, Splicingvarianten und posttranslational modifizierten Formen von DHRS4 mit DHRS4-Immunreaktivität in einer biologischen Flüssigkeit, insbesondere Serum oder Plasma, eines Patienten.

3. *Ex vivo* Verfahren zur differentialdiagnostischen Früherkennung und Erkennung, für die Verlaufsprognose und die Beurteilung des Schweregrads und zur Verlaufs- und Therapiekontrolle von Sepsis und schweren Infektionen, insbesondere sepsisähnlichen systemischen Infektionen, **dadurch gekennzeichnet, dass** man in einer biologischen Flüssigkeit eines Patienten mit Hilfe eines Ligandenbindungsassays die Anwesenheit und/oder Menge von kurzkettiger SRL-Alkolioldehydrogenase (DHRS4) mit der Aminosäuresequenz gemäß SEQ ID NO:1 und/oder von pathophysiologisch auftretenden Fragmenten, Splicingvarianten und posttranslational modifizierten Formen von DHRS4 mit DHRS4-Immunreaktivität bestimmt und aus dem Nachweis und/- oder der Menge der DHRS4-Immunreaktivität Schlüsse hinsichtlich des Vorliegens, des zu erwartenden Verlaufs, des Schweregrads oder des Erfolgs eines Therapie einer Sepsis oder Infektion zieht.

4. Verfahren nach Anspruch 3, **dadurch gekennzeichnet, dass** der Ligandenbindungsassay ein heterogener oder homogener Immunoassay in Form eines Sandwich-Assays ist.

5. Verfahren nach einem der Ansprüche 3 oder 4, **dadurch gekennzeichnet, dass** es im Rahmen einer Multiparameter-Bestimmung durchgeführt wird, bei der gleichzeitig mindestens ein weiterer Sepsisparameter bestimmt wird und ein Messergebnis in Form eines Satzes von mindestens zwei Messgrößen gewonnen wird, der zur Sepsis-Feindiagnostik und zur Patienten-Stratifizierung ausgewertet wird.

6. Verfahren nach Anspruch 5, **dadurch gekennzeichnet, dass** im Rahmen der Multiparameter-Bestimmung neben DHRS4-Immunreaktivität wenigstens ein weiterer Parameter bestimmt wird, der ausgewählt ist aus der Gruppe, die besteht aus Procalcitonin, CA 19-9, CA 125, S100B, S100A-Proteinen, löslichen Cytokeratin-Fragmenten, insbesondere CYFRA 21, TPS und/oder löslichen Cytokeratin-1-Fragmenten (sCY1F), den Enzymen Aldose-1-Epimerase, Glycin-N-Acyl-Transferase (GNAT), Cu/Zn-SOD, Carbamoylphosphat-Synthetase (CPS) und Fragmenten der genannten Enzyme, den Peptiden Inflammin, CHP, LASP-1, Gastrokine 1 sowie Immunreaktivität, die sich ableitet Vorläufern von vasoaktiven Peptiden, insbesondere von Pro-ANP, Pro-Endothelin, Pro-Vasopressin und Pro-Adrenomedullin sowie Cytokinen, Interleukinen, TNF und dem C-reaktiven Protein (CRP).

7. Verfahren nach Anspruch 5 oder 6, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

8. Verfahren nach Anspruch 7, **dadurch gekennzeichnet, dass** die Auswertung des mit der Messvorrichtung gewonnenen komplexen Messergebnisses mit Hilfe eines Computerprogramms erfolgt.

9. Verfahren nach Anspruch einem der Ansprüche 3 bis 4, **dadurch gekennzeichnet, dass** es als Schnelltest oder POC (Point-of-Care) Test ausgestaltet ist.

10. Verfahren nach Anspruch 9, **dadurch gekennzeichnet, dass** es ein immunchromatographisches Verfahren mit einer visuell erkennbaren Direktmarkierung ist.

## Claims

1. Use of short-chain SRL alcohol dehydrogenase (DHRS4) having the amino acid sequence according to SEQ ID No: 1 as a humoral biomarker for diagnostic detection and for prognosis and monitoring of the course and the therapy of inflammations and infections.

2. Use according to Claim 1 in differential diagnostic early detection and detection, for determination of the severity and for monitoring the course and the therapy of sepsis and severe infections by ex vivo determination of the occurrence and/or of the amount of DHRS4 and/or of pathophysiologically occurring fragments, splicing variants and post-translationally modified forms of DHRS4 with DHRS4 immunoreactivity in a biological fluid, in particular serum or plasma, of a patient.

3. Ex vivo method for differential diagnostic early detection and detection, for prognosis and assessment of the severity and for monitoring the course and therapy of sepsis and severe infections, in particular sepsis-like systemic infections, **characterized in that** the presence and/or amount of short-chain SRL alcohol dehydrogenase (DHRS4) having the amino acid sequence according to SEQ ID NO:1 and/or of pathophysiologically occurring fragments, splicing variants and post-translationally modified forms of DHRS4 with DHRS4 immunoreactivity are determined in a biological fluid of a patient with the aid of a ligand-binding assay, and conclusions about the presence, the expected course, the severity or the success of a therapy of a sepsis or infection are drawn from the detection and/or the amount of DHRS4 immunoreactivity.

4. Method according to Claim 3, **characterized in that** the ligand-binding assay is a heterogeneous or homogeneous immunoassay in the form of a sandwich assay.

5. Method according to any of Claims 3 or 4, **characterized in that** it is carried out as part of a multi-parameter determination in which at the same time at least one further sepsis parameter is determined and a measured result in the form of a set of at least two measured variables is obtained, which is evaluated for fine sepsis diagnosis or for patient stratification.

6. Method according to Claim 5, **characterized in that**, as part of the multi-parameter determination, at least one further parameter which is selected from the group which consists of procalcitonin, CA19-9, CA125, S100B, S100A proteins, soluble cytokeratin fragments, in particular CYFRA21, TPS and/or soluble cytokeratin-1 fragments (sCY1F), the enzymes aldose-1-epimerase, glycine N-acyl transferase (GNAT), Cu/Zn-SOD, carbamoyl phosphate synthetase (CPS) and fragments of said enzymes, the peptides inflammin, CHP, LASP-1, gastrokine 1 and immunoreactivity which is derived from precursors of vasoactive peptides, in particular of pro-AMP, pro-endothelin, 4-vasopressin and pro-adrenomedullin, and cytokines, interleukins, TNF and the C-reactive protein (CRP) is determined in addition to DHRS4 immunoreactivity.

7. Method according to Claim 5 or 6, **characterized in that** the multi-parameter determination is effected as a simultaneous determination by means of a chip technology measuring apparatus or an immunochromatographic measuring apparatus.

8. Method according to Claim 7, **characterized in that** the evaluation of the complex measured result obtained with the measuring apparatus is effected with the aid of a computer program.

9. Method according to any of Claims 3 to 4, **characterized in that** it is designed as a quick test or POC (point-of-care) test.

10. Method according to Claim 9, **characterized in that** it is an immunochroinatographic method with a visually detectable direct marking.

## Revendications

1. Utilisation de déshydrogénase d'alcool SRL (DHRS4) de chaîne courte, avec la séquence d'acides aminés selon SEQ ID NO : 1, en tant que marqueur biologique humoral pour le diagnostic et pour le pronostic de l'évolution ainsi que pour le contrôle de l'évolution et de la thérapie d'inflammations et d'infections.

2. Utilisation selon la revendication 1 dans le cadre de la détection précoce et du diagnostic différentiels pour la détermination du degré de gravité et pour le contrôle de l'évolution et de la thérapie de la septicémie et de graves infections, par la détermination ex vivo de l'apparition et / ou de la quantité de DHRS4 avec la séquence d'acide aminés selon SEQ ID NO : 1, et / ou de fragments se manifestant de manière physiologique pathologique, de variantes d'épissage et de formes de DHRS4 modifiées au niveau de la posttraduction, avec immunoréactivité DHRS4 dans un liquide biologique, en particulier sérum ou plasma, d'un patient.

3. Procédé ex vivo pour la détection précoce et le diagnostic différentiels, pour le pronostic de l'évolution et l'évaluation du degré de gravité, et pour le contrôle de l'évolution et de la thérapie de la septicémie et de graves infections, en particulier d'infections systémiques septicémiques, analogues à la septicémie, **caractérisé en ce que** l'on détermine, dans un liquide biologique d'un patient, au moyen d'un essai de liaison de coordination, la présence et / ou la quantité de déshydrogénase d'alcool SRL (DHRS4) de chaîne courte, avec la séquence d'acides aminés selon SEQ ID NO : 1 et / ou de fragments se manifestant de manière physiologique pathologique, de variantes d'épissage et de formes de DHRS4 modifiées au niveau de la posttraduction, avec immunoréactivité DHRS4, et tire du décèlement et / ou de la quantité de l'immunoréactivité DHRS4 des conclusions en ce qui concerne la présence, l'évolution attendue, le degré de gravité ou le succès d'une thérapie d'une septicémie ou d'une infection.

4. Procédé selon la revendication 3, **caractérisé en ce que** l'essai de liaison de coordination est un immunoessai hétérogène ou homogène, sous la forme d'un essai sandwich.

5. Procédé selon l'une des revendications 3 et 4, **caractérisé en ce qu'**il est exécuté dans le cadre d'une détermination multiparamètre, dans laquelle au moins un autre paramètre de septicémie est simultanément déterminé, et un résultat est obtenu sous la forme d'un jeu d'au moins deux valeurs de mesure, qui est exploité pour le diagnostic précis de la septicémie et pour la stratification du patient.

6. Procédé selon la revendication 5, **caractérisé en ce que**, dans le cadre de la détermination multiparamètre, est déterminé, à côté de l'immunoréactivité DHRS4, au moins un autre paramètre, qui est sélectionné dans le groupe qui comprend la procalcitonine, CA 19-9, CA 125, S100B, les protéines S100A, des fragments de cytokératine solubles, en particulier CYFRA 21, TPS, et / ou des fragments de cytokératine 1 (sCY1F) solubles, les enzymes aldose-1-épimérase, les transférases de glycine-N-Acyle (GNAT), SOD Cu / Zn, synthétase de phosphate carbamoylique (CPS) et des fragments des enzymes cités, les peptides inflammine, CHP, LASP-1, gastrokine 1, ainsi que l'immunoréactivité, les précurseurs dérivés de peptides vasoactifs, en particulier de pro-ANP, de proendothéline, de pro-vasopressine et de proadrénomédulline, ainsi que cytokines, interleukines, TNF et la protéine C réactive (CRP).

7. Procédé selon revendication 5 ou 6**, caractérisé en ce que** la détermination multiparamètre est effectuée en tant que détermination simultanée au moyen d'un dispositif de mesure selon la technologie des microplaquettes ou d'un dispositif de mesure immunochromatographique.

8. Procédé selon la revendication 7, **caractérisé en ce que** l'évaluation du résultat de mesure complexe, obtenu avec le dispositif de mesure, est effectuée à l'aide d'un programme d'ordinateur.

9. Procédé selon l'une des revendications 3 à 4, **caractérisé en ce qu'**il est conçu en tant que test immédiat ou en tant que test PC (Point-of-Care).

10. Procédé selon la revendication 9, **caractérisé en** celui-ci est un procédé immunochromatographique avec un marquage direct, visuellement discernable.
